# EUROPEAN PATENT APPLICATION

(11) **EP 4 696 342 A1**
(43) Date of publication of application: **18.02.2026**
(21) Application number: 25196255.1
(22) Date of filing: 15.08.2025
(51) Int. Cl.: A61M 1/02, A61M 1/38, A61M 39/18

(54) **SYSTEM AND METHOD FOR DETECTION AND IDENTIFICATION OF TUBING ASSOCIATED WITH A MEDICAL DEVICE**

(30) Priority: 16.08.2024 US 202463684097 P
(71) Applicant: Fenwal, Inc., Lake Zurich, IL 60047 (US)
(72) Inventor: KUSTERS, Benjamin E., Lake Zurich, 60047 (US); MADSEN, James, Lake Zurich, 60047 (US)
(74) Representative: Maikowski & Ninnemann Patentanwälte Partnerschaft mbB

(57) **Abstract**

Systems and methods for ensuring the proper placement of color-coded tubing on a medical device are disclosed. The system includes one or more sensors and a computer memory containing a database of acceptable colors associated with said one or more sensors. The system is configured to compare the measured color of said color-coded tubing to colors of a color database, wherein the sensor is associated with a color from said database.

## Description

### Field of the Disclosure

The present disclosure is directed to systems and methods for mating a disposable fluid circuit and a reusable hardware device. More particularly, the present disclosure is directed to a systems and methods for determining if a disposable tubing kit is loaded in the correct location on an associated hardware device. The methods and systems described herein are particularly useful in the medical field such as in the field of blood/biological fluid processing and in the assembly of disposable fluid circuits used in such processing.

### Background

Biological fluid and/or blood processing systems often utilize a reusable hardware device and a disposable tubing kit that is mounted/loaded onto the reusable hardware device in a pre-determined mating arrangement. Many such systems are able to detect the presence of tubing and confirm that the kit has been mounted, but are not able to identify and/or differentiate tubing sections to ensure that the disposable kit is properly installed and that the correct tubing or tubing portion is present in the correct location on the device. For example, some existing blood processing systems may aid in the loading of tubing by providing color-coded tubing guides as visual cues for the user during the loading of the tubing kit. One such system is the Amicus Separator, available from Fresenius Kabi of Bad Homburg, Germany. The Amicus Separator may be used with a variety of disposable tubing kits for blood collection and therapeutic procedures, each requiring loading in a pre-determined configuration.

In addition to providing a visual cue for loading a disposable kit onto a reusable hardware device, currently available systems may also employ kit pressure checks to further ensure proper kit loading. Such pressure checks typically employ pressure sensors and pumps to generate pressures within the kit tubing. However, not all devices used in the field of blood or biological fluid processing utilize pressure sensors or pumps for generating pressures. Some devices, such as sterile connection devices, are utilized in the assembly of tubing, but not in the actual processing applications.

Thus, it would be desirable to provide systems and methods that allow for quickly and reliably detecting the presence of tubing and identifying the tubing to ensure that the tubing is in the correct location on the device.

### Summary

In one aspect, the present disclosure is directed to a system for ensuring the proper placement of medical tubing on a reusable hardware device. The system includes a device configured to receive color-coded tubing for mounting thereon, the device including one or more sensors and a controller coupled to the one or more sensors. The device includes a computer memory containing a database of acceptable colors associated with the one or more sensors. The system is configured to generate an alert when a sensor of the one or more sensors detects a color other than an acceptable color for the sensor. The system also includes the color-coded tubing mounted on the device in proximity to said one or more sensors.

In another aspect, the present disclosure is directed to a method for detecting the proper placement of tubing on a reusable hardware device. The method includes placing color-coded tubing in proximity to a colorimetric optical sensor located at a selected location on the device. The method further includes detecting the presence of tubing at the selected location and measuring the color of the color-coded tubing at the selected location. The method further includes comparing the measured color of said tubing to colors of a color database, wherein the sensor is associated with a color from said database. The method also includes determining if the measured color of the tubing matches the color associated with the sensor located at the selected location.

### Brief Description of the Drawings

Figure 1 is a perspective view of a device onto which medical tubing may be mounted;
Figure 2 is an enlarged view of an exemplary sensor and tubing arrangement in accordance with the present disclosure;
Figure 3 is a perspective view of an example of a color-coded tubing segment used in the methods and system of the present disclosure;
Figure 4 is a perspective view of another example of a color-coded tubing segment used in the methods and system of the present disclosure; and
Figure 5 is flow-chart depicting the system logic of the method of the present disclosure.

### Detailed Description of the Embodiments

A more detailed description of the systems and methods in accordance with the present disclosure is set forth below. It will be understood that the description below of specific devices and methods is intended to be exemplary, and not exhaustive of all possible variations or applications.

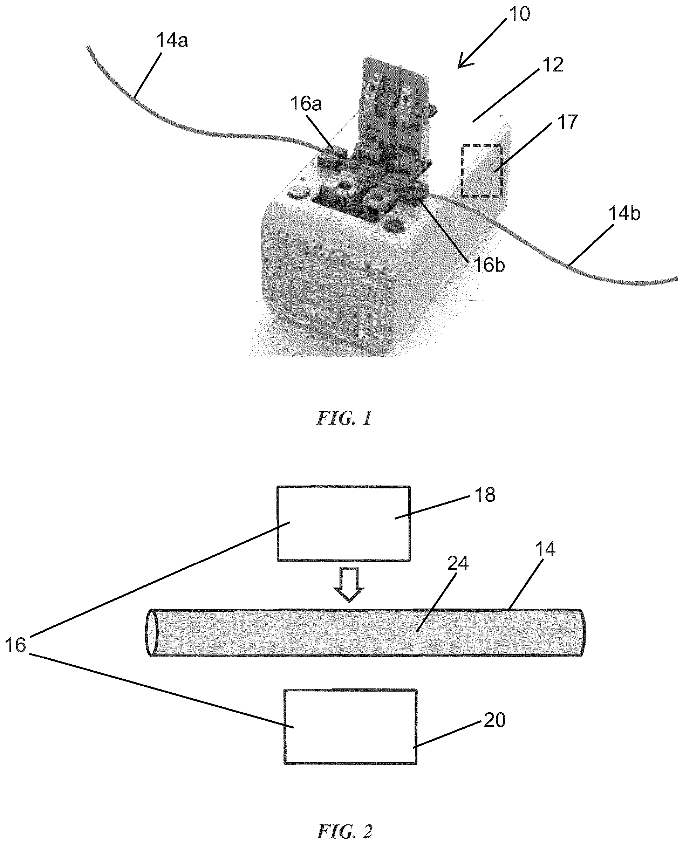

Fig. 1 depicts a system 10 that includes a reusable hardware device 12 and disposable tubing 14 that is mounted onto the device. In the particular embodiment shown in Fig. 1, device 12 is a sterile connection device used to join medical tubing segments in a sterile manner. Of course, it will be understood that other devices and tubing may utilize the systems and methods disclosed herein, including without limitation, apheresis systems, cell processing and cell therapy systems, blood pack units (BPUs) for the manual collection of blood and blood components and the like.

In the device of Fig. 1, tubing segments 14a and 14b are mounted in proximity or within a sensor subassembly 16a or 16b which may include a light source 18 and a receiver 20. In accordance with the present disclosure, the sensor is preferably a colorimetric optical sensor which is capable of detecting the color of an object located within the sensor subassembly 16. The colorimetric optical sensor may be configured for various color measurements including transmission and reflection-based sensors or spectrometers capable of measuring the dominant wavelength of color using a color specification system, such as the CIE 1931 color space.

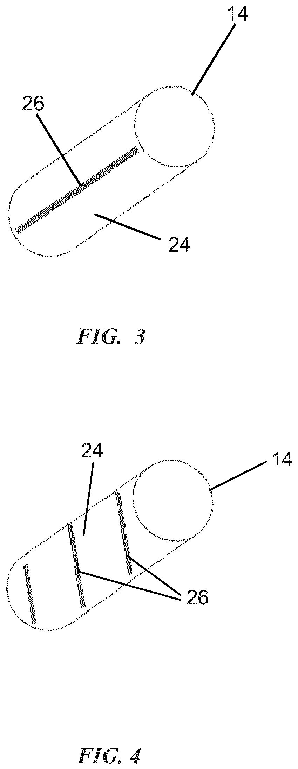

Sensors 16a and 16b may be located anywhere where tubing identification is required or desired. In the example of Fig. 1, two sensors16a and 16b, one for each of tubing segments 14a and 14b are shown, but it will be understood that in other devices and other applications, the number of sensors may be more or less than two. Sensors 16 (16a and 16b) may be coupled to a device controller 17 or include an internal control system that receives readings from sensor subassembly 16. Controller 17 or control system may include a computer memory that stores a database of colors associated with each of the sensors 16a, 16b of device 12. Sensors 16a, 16b may be configured to detect the overall color of tubing 14a, 14b and more particularly the color of tubing outer wall 24. In another embodiment, tubing 14a, 14b may include a different color identifier such as a colored line or spiral as shown in in Figs. 3 and 4, or any other colored marking readable and detectable by sensors 16a, 16b.

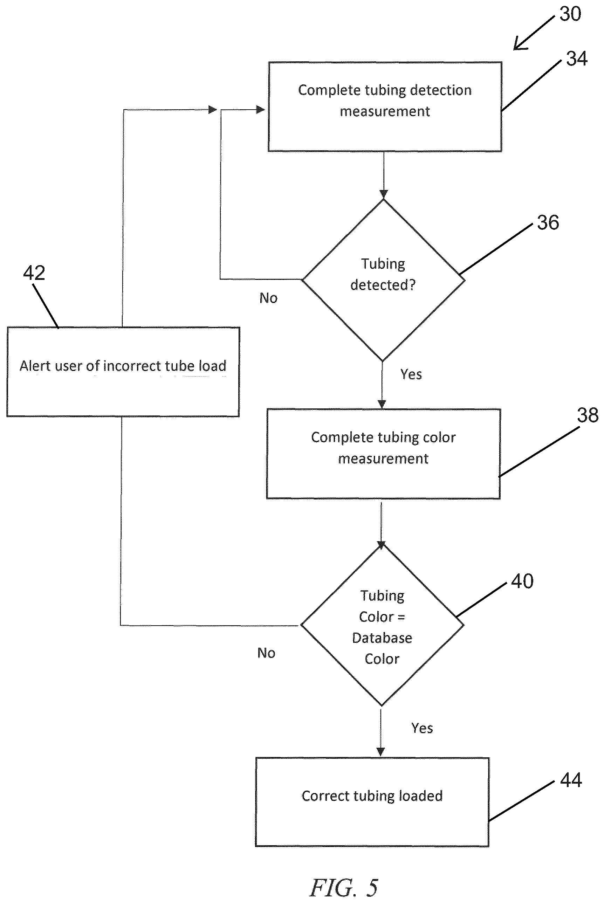

In addition to detecting colors of the tubing associated therewith, sensors 16a, 16b also detect the presence of tubing in proximity to or inserted within sensor subassembly16. Thus, with reference to Fig. 5 and in accordance with the method 30 of ensuring correct placement of tubing 14, optical sensors 16a, 16b are configured to first detect the presence of the tubing. Once the presence of tubing has been detected and confirmed (step 34), system 30 measures the color associated with the tubing (step 36). In an embodiment, the tubing color measurement of a specific color may likewise serve as a detection of the presence of tubing. The sensor may measure the color of the tubing, or any other means of color identification associated with a tubing as shown in Figs. 3 and 4 and described above. Once the sensor detects the tubing and identifies the color associated with the tubing (whether in two steps or in one step as described above), the color identification is compared to a database of acceptable colors associated with the sensor (step 40). The database may be configured depending on the type of device process selected by a user. For example, in Figure 1, sensor 16a detects the measured color of tubing 14a and compares the measured color to the acceptable database color to ensure that the loaded tubing color matches the color associated with sensor 16a. Sensor 16b compares the measured color of tubing 14b to the acceptable database color to ensure the tubing color likewise matches the color associated with sensor 16b. This method would be completed for all sensors present on a device for identification of tubing through the proposed color-based method. If the tubing color measured by a sensor does not match an acceptable color from the database, the system may alert the operator (step 42) and prevent the device from proceeding with the intended procedure until the tube loading error is corrected.

### Other Examples

There are additional aspects to the systems and methods described herein including, without limitation, the following Aspects.

Aspect 1: A system for ensuring the proper placement of tubing on a device including a reusable hardware device configured to receive tubing for mounting thereon. The device including one or more sensors and a controller coupled to the one or more sensors and including a computer memory containing a database of acceptable colors associated with said one or more sensors. The device is configured to generate an alert when a sensor of the one or more sensors detects a color other than an acceptable color for the sensor. The system also includes a color-coded tubing mounted on the device in proximity to the one or more sensors.

Aspect 2: The system of Aspect 1 wherein the one or more sensors includes a colorimetric optical sensor.

Aspect 3: The system of Aspect 2 wherein the colorimetric optical sensor is selected from the group consisting of (a) transmission and reflection-based sensor and (b) spectrometers capable of measuring the dominant wavelength of color.

Aspect 4: The system of any one of Aspects 1 through 3 wherein the one or more sensors is configured to detect the presence of tubing.

Aspect 5: The system of any one of Aspects 1 through 4 including at least two sensors wherein each of the two sensors is associated with separate pieces of tubing wherein the separate pieces of tubing are configured to be joined together.

Aspect 6: The system of any one pf Aspects 1 through 5 wherein the tubing is part of a disposable fluid circuit configured for mounting on a medical device.

Aspect 7: The system of any one of Aspects 1 through 6 wherein the tubing is made from a polymeric material wherein the tubing includes an outer wall surface at least a portion of which includes a color different from a color of the polymeric material from which the tubing is made.

Aspect 8: The system of Aspect 7 wherein the outer wall surface of the tubing includes a marking in a detectable color.

Aspect 9: The system of Aspect 8 wherein the marking comprises a line on the outer wall surface of the tubing.

Aspect 10: The system of any one of Aspects 1 through 9 wherein each of the one or more sensors is associated with a specific location on said device.

Aspect 11: The system of Aspect 10 wherein the device is configured to generate one or both of an audible and visual alert when a sensor detects a tubing color that is not associated with the specific location.

Aspect 12: The system of Claim 11 wherein the device prevents further operation of the device when an alert is generated.

Aspect 13: The system of any one of Aspects 1 through 12 wherein the device comprises a sterile connection device.

Aspect 14: The system of any one of Aspects 1 through 12 wherein said device comprises a biological fluid processing device.

Aspect 15: A method for detecting the proper placement of tubing on a device including placing a color-coded tubing in proximity to a colorimetric optical sensor located at a selected location on a device; detecting the presence of tubing at said selected location; measuring the color of the color-coded tubing at the selected location; comparing the measured color of the tubing to colors of a color database, wherein the sensor is associated with a color from said database; and determining if the measured color of the tubing matches the color associated with the sensor located at the selected location.

Aspect 16: The method of Aspect 15 further including preventing further operation of the device if the measured color does not match the color associated with the sensor located at the selected location.

Aspect 17: The method of Aspect 16 including generating an alert if the measured color does not match the color associated with the sensor located at the selected location.

Aspect 18: The method of Aspect 15 including generating a signal that the measured color matches the color associated with the sensor located at the selected location.

Aspect 19: The method of any one of Aspects 15 through 18 wherein the medical device comprises a sterile connection device.

Aspect 20: The method of any one of Aspects 15 through 19 wherein detecting the presence of tubing and measuring the color of the color coded occurs simultaneously.

It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

## Claims

1. A system for ensuring the proper placement of tubing on a device comprising:
a) a reusable hardware device configured to receive tubing for mounting thereon, said device including one or more sensors and a controller coupled to said one or more sensors, said device comprising a computer memory containing a database of acceptable colors associated with said one or more sensors, said wherein said device is configured to generate an alert when a sensor of said one or more sensors detects a color other than an acceptable color for said sensor;
b) color-coded tubing mounted on said device in proximity to said one or more sensors.

2. The system of Claim 1 wherein said one or more sensors comprises a colorimetric optical sensor, preferably wherein said colorimetric optical sensor is selected from the group consisting of (a) transmission and reflection-based sensor and (b) spectrometers capable of measuring the dominant wavelength of color.

3. The system of any one of Claims 1 through 2 wherein said one or more sensors is configured to detect the presence of tubing.

4. The system of any one of Claims 1 through 3 comprising at least two sensors wherein each of said two sensors is associated with a separate pieces of tubing wherein said separate pieces of tubing are configured to be joined together.

5. The system of any one pf Claims 1 through 4 wherein said tubing is part of a disposable fluid circuit configured for mounting on a medical device.

6. The system of any one of Claims 1 through 5 wherein said tubing is made from a polymeric material wherein said tubing comprises an outer wall surface at least a portion of which comprises a color different from a color of the polymeric material from which said tubing is made.

7. The system of Claim 6 wherein said outer wall surface of said tubing includes a marking in a detectable color, preferably wherein the marking comprises a line on the outer wall surface of said tubing.

8. The system of any one of Claims 1 through 7 wherein each of said one or more sensors is associated with a specific location on said device.

9. The system of Claim 8 wherein said device is configured to generate one or both of an audible and visual alert when a sensor detects a tubing color that is not associated with said specific location, preferably wherein said device prevents further operation of said device when an alert is generated.

10. The system of any one of Claims 1 through 9 wherein said device comprises a sterile connection device or wherein said device comprises a biological fluid processing device.

11. A method for detecting the proper placement of tubing on a device comprising:
a. placing a color-coded tubing in proximity to a colorimetric optical sensor located at a selected location on a device;
b. detecting the presence of tubing at said selected location;
c. measuring the color of said color-coded tubing at said selected location;
d. comparing the measured color of said tubing to colors of a color database, wherein said sensor is associated with a color from said database; and
e. determining if said measured color of the tubing matches the color associated with the sensor located at the selected location.

12. The method of Claim 11 further comprising preventing further operation of said device if said measured color does not match the color associated with the sensor located at said selected location and preferably comprising generating an alert if said measured color does not match the color associated with the sensor located at said selected location.

13. The method of Claim 11 comprising generating a signal that said measured color matches the color associated with the sensor located at the selected location.

14. The method of any one of Claims 11 through 13 wherein the medical device comprises a sterile connection device.

15. The method of any one of Claims 11 through 14 wherein said detecting the presence of tubing and said measuring the color of said color coded occurs simultaneously.
